# EUROPEAN PATENT APPLICATION

(11) **EP 4 299 050 A1**
(43) Date of publication of application: **03.01.2024**
(21) Application number: 22305987.4
(22) Date of filing: 01.07.2022
(51) Int. Cl.: A61K 8/34, A61K 8/37, A61K 8/92, A61Q 19/10

(54) **SEMISOLID CLEANSING COMPOSITIONS**

(71) Applicant: Johnson & Johnson Consumer Inc., Skillman, NJ 08558 (US)
(72) Inventor: CANDOLIVES, Marilyne, 27110 SAINTE-OPPORTUNE-DU-BOSC (FR); BOUCHER, Amélie, 27430 ST ETIENNE DU VAUVRAY (FR); GUYOMARD, Alexandre, 75013 PARIS (FR)
(74) Representative: Carpmaels & Ransford LLP

(57) **Abstract**

A cleansing composition in the form of a balm, comprising: a naturally occurring wax, wherein the wax comprises at least about 10% by weight of the cleansing composition of cetearyl alcohol, and at least about 6.5% by weight of the cleansing composition of either hydrogenated castor oil or cera alba or a mixture thereof; an oil, the oil being liquid at 20 °C, wherein at least about 50% by weight of the oil is caprylic/capric triglyceride or isopropyl myristate or a mixture thereof; a surfactant; and optionally an emollient, wherein the cleansing composition is substantially free of water.

## Description

### Field of the Invention

The present invention relates to improved cleansing compositions, for use in cleansing skin surfaces. In particular, the invention is useful in cleansing the facial areas of the skin, and is particularly useful in cleansing various makeup products.

### Background of the Invention

Cleansers come in many different forms, it may be useful to have a cleanser that is capable of removing dirt, oil, grease, or makeup, in the form of a balm. In particular, it is helpful to have a cleansing balm that does not include any synthetic wax. The cleansing balm should be aesthetically pleasing and comfortable to apply to the skin, and is desirably anhydrous. When the product is rinsed off the skin, the product should easily emulsify, allowing simple removal of the product, as well as the dirt, oil, grease, or makeup, from the skin.

Further, the cleansing balm may be stable at elevated temperatures, e.g., up to 50 °C, without melting or formation of crystals or droplets therein. Prior makeup cleansers lose stability at elevated temperatures, including those temperatures that may be experienced in summer months. These unstable cleansers may become liquid, and if the container in which it is contained is not stored properly, can leak and spill. Further, the lack of stability can cause undesired aesthetics, such as droplets or crystals forming.

The present invention provides a cleansing composition in the form of a balm that satisfies the aforementioned criteria.

### Summary of the Invention

The present invention includes an improved cleansing composition. The cleansing composition is in the form of a balm comprising a naturally occurring wax, wherein the naturally occurring wax comprises at least about 10% by weight of the cleansing composition of cetearyl alcohol, and at least about 6.5% by weight of the cleansing composition of either hydrogenated castor oil or cera alba or a mixture thereof; an oil, the oil being liquid at 20 °C, wherein at least about 50% by weight of the oil is caprylic/capric triglyceride or isopropyl myristate or a mixture thereof; a surfactant; and optionally an emollient. The cleansing composition is substantially free of water. The cleansing composition preferably has a hardness value of from about 25 grams to about 125 grams at 20 °C, which is desirable for maintaining the cleansing composition in the form of a balm prior to use.

The present invention further provides the use of a cleansing composition as defined herein to remove at least one of dirt, oil, grease or makeup from skin.

The present invention further provides a method of cleansing skin comprising
(i) applying the cleansing composition as defined herein to a region of skin of a user;
(ii) subsequently applying water to the region of skin, thereby causing the cleansing composition to emulsify; and
(iii) rinsing the region of skin to remove the emulsified cleansing composition.

### Detailed Description of the Invention

The present invention is directed to a cleansing composition; the use of cleansing compositions to remove dirt, oil, grease and makeup from skin; and methods of cleansing skin. In particular, the cleansing composition is in the form of a balm (also described herein as a 'cleansing balm'), which is defined herein as being a substantially nonaqueous, semisolid material. Preferably, the cleansing composition has a hardness value described in greater detail below. The cleansing composition in the form of a balm is capable of being stored in a suitable container, where it maintains a desired semisolid state prior to being removed from the container and applied to the skin by the user. When applied to the skin, the balm attracts and removes unwanted materials such as dirt, oil, grease, or makeup from the skin. The balm is capable of quickly and easily forming an emulsion when mixed with water, thereby allowing it to be rinsed off from the skin efficiently and safely, taking with it the attracted dirt, oil, grease, or makeup. In one exemplary method of using the balm, the user may remove some of the balm from its initial container, such as with the user's finger, and apply it to the desired areas of the skin or face. Thereafter, the user applies water to the region of the skin or face to which the balm has been applied, which causes the balm to emulsify, and the emulsified balm may be removed by rinsing. It is desired that the balm be removed from the skin within five minutes after applying to the skin, or within three minutes after applying to the skin, or within one minute after applying to the skin. The balm may be removed from the skin after a period of time, wherein the period of time is from about 5 seconds to about 5 minutes, or from about 10 seconds to about 3 minutes, or from about 20 seconds to about 1 minute. The cleansing composition is desirably mild enough to be applied to the face of a user without stinging or causing irritation to the skin, particularly near the ocular area of the face.

The present invention provides cleansing compositions that deliver optimal cleansing properties. As used herein, the term "optimal" refers to and includes comparable and/or improved cleansing efficacy when compared to known cleansers. Optimal does not necessarily mean 100% cleansing efficiency, but rather is intended to mean a suitable efficiency to remove various materials, such as dirt, oil, grease, makeup or cosmetics from the skin and face. Optimal and/or efficient cleansing is described below in greater detail for various cosmetics.

Other benefits provided by the cleansing compositions of the present invention may include one or more of the following: visual and skin feel aesthetics, stability in a semisolid state at room temperature and at elevated temperatures, reduced greasiness, low irritation of skin and eyes, achieving shorter application time to the skin with low force required, reducing the amount of cleansing agents in the composition, and enabling the cleansing compositions to be free of silicone and/or antimicrobial preservatives. The cleansing composition is stable at room temperature and at elevated temperatures, may be free of undesired cracks, droplets and/or crystals after formation, and has a desired hardness value prior to use, described below. The product is capable of being emulsified in the presence of water quickly and efficiently, allowing it to be rinsed from the skin after application and mixing with water.

As used herein, the term "percent" shall refer to the weight percent, and similarly, all amounts set forth herein shall be by weight unless noted otherwise. The term "substantially" when used herein, shall mean within 1%, or within 0.5%, or within 0.1%. By way of example, the term "substantially free" of a material means less than 1%, less than 0.5%, or less than 0.1%, or less than 0.05% of the material by weight (e.g., the term "substantially nonaqueous" means less than 1%, less than 0.5%, or less than 0.1%, or less than 0.05% of aqueous material by weight). The term "substantially all" of a material means greater than 99%, greater than 99.5% or greater than 99.9% of the material by weight.

The cleansing composition of the present invention is desirably a substantially anhydrous material prior to use. The cleansing composition is substantially free of water. The cleansing composition may have less than about 1% water by weight of the cleansing composition, or less than about 0.5% water, or less than about 0.1% water, or less than about 0.05% water. It may be desired that the composition is free of any added water, although it is understood that ambient moisture in the air or trace water in an ingredient (such as may occur with panthenol) may add a minor amount of water to the composition.

Generally, the present invention includes cleansing compositions further comprising one or more of the following: humectant(s), preservative(s), fragrance(s), excipient(s), extract(s), and buffer(s). Various combinations of the foregoing components are useful in the present invention.

A cleansing composition may include one or more antioxidants. The cleansing composition may include one or more antioxidants, one or more humectants, and optionally may include a fragrance ingredient or fragrance ingredient blends. The cleansing composition may be free or substantially free of antimicrobial preservatives.

The cleansing compositions useful in the present invention may comprise an emollient. The cleansing compositions useful in the present invention may be formulated comprising a combination of at least two emollients. As used herein, "emollients" refer to materials used for cleansing the skin, hair, and eye lashes, the prevention or relief of dryness, or for the protection of the skin. Nonlimiting examples of emollients include hydrophobic compounds such as vegetable oils, mineral oils (e.g., petrolatum), fatty esters (e.g., isopropyl palmitate, C12-C15 alkyl benzoate) including those fatty esters of glycerol, and the like.

The cleansing compositions of the present invention are in the form of a balm. A balm is considered "stable" if it remains semisolid prior to use, desirably having a hardness value of from about 25 grams to about 125 grams at 20 °C, as measured in accordance with the hardness value measurement method described below. The hardness value may be from about 25 grams to about 60 grams, or from about 25 grams to about 50 grams at 20 °C. Other suitable hardness values at 20°C include a hardness value of from about 122 grams to about 124 grams, or about 107 grams to about 109 grams, or from about 56 grams to about 58 grams, or from about 42 grams to 44 grams, or from about 27 grams to 29 grams, or about 123 grams, or about 108 grams, or about 57 grams, or about 28 grams, or about 43 grams. In addition, the balm should also lack visible crystals or droplets prior to use, and desirably avoids cracking in the absence of force applied thereon. The balm desirably remains stable in its container for at least six months or at least one year. Lack of stability may be evidenced by the formation of crystals or droplets in the balm, which are visible to the eye without the need for a microscope. Lack of stability may also or alternatively be evidenced by melting at room temperature. Lack of stability may also or alternatively be evidenced by formation of cracks in the balm, where the cracks are not the result of force applied to the balm. The present inventive cleansing composition is stable at room temperature (e.g., about 20 °C), and may be stable at elevated temperatures, as described herein. Elevated temperatures include temperatures up to 30 °C, up to 40 °C, or up to 50 °C. Being stable at various temperature may be beneficial in some aspects, since the balm may be used in different geographic regions without the need for being kept in a chilled or cooled environment. Further, if a balm is stable at elevated temperatures, it can withstand warmer rooms or environments, including warm or sunny days, which is particularly useful to consumers.

As mentioned above, the cleansing composition of the present invention includes a naturally occurring wax, wherein the naturally occurring wax comprises at least about 10% by weight of the cleansing composition of cetearyl alcohol, and at least about 6.5% by weight of the cleansing composition of either hydrogenated castor oil or cera alba (also referred to as beeswax) or a mixture thereof. Typically, the cleansing composition is free of or substantially free of synthetic wax, such as cera microcristallina. The naturally occurring wax may further comprise glyceryl stearate, or cetearyl olivate, or sorbitan olivate, or glyceryl behenate, or combinations thereof. The total amount of naturally occurring wax present in the composition may be from about 16.5% to about 50% by weight of the cleansing composition, or from about 16.5% to about 40% by weight of the cleansing composition, or from about 20% to about 30% by weight of the cleansing composition, or from about 21% to about 39% by weight of the cleansing composition.

Cetearyl alcohol may be present in an amount of at least about 12% by weight of the cleansing composition, or at least about 14% by weight of the cleansing composition. Cetearyl alcohol may be present in an amount of from about 10% to about 30% by weight of the cleansing composition, or from about 10% to about 25% by weight of the cleansing composition, preferably about 25% by weight of the cleansing composition. When cera alba is used, it may be present in an amount of at least about 6.5% by weight of the cleansing composition, or at least about 10% by weight of the cleansing composition, or at least about 12% by weight of the cleansing composition. Cera alba may be present in an amount of from about 6.5% to about 30% by weight of the cleansing composition, or from about 10% to about 14% by weight of the cleansing composition, preferably about 12% by weight of the cleansing composition. When hydrogenated castor oil is used, it is desired that the hydrogenated castor oil be present in an amount of at least about 6.5% by weight of the cleansing composition, or at least about 9% by weight of the cleansing composition. Hydrogenated castor oil may be present in an amount of from about 6.5% to about 30% by weight of the cleansing composition, or from about 9% to about 25% by weight of the cleansing composition. When a mixture of cera alba and hydrogenated castor oil is used, it may be present in an amount of at least about 8% by weight of the cleansing composition, or at least about 10% by weight of the cleansing composition, or at least about 12% by weight of the cleansing composition. A mixture of cera alba and hydrogenated castor oil may be present in an amount of from about 6.5% to about 30% by weight of the cleansing composition, or from about 8% to about 25% by weight of the cleansing composition, or from about 10% to about 14% by weight of the cleansing composition.

The cleansing composition may include a combination of cetearyl alcohol and cera alba, or it may include a combination of cetearyl alcohol and hydrogenated castor oil, or it may include a combination of cetearyl alcohol, cera alba and hydrogenated castor oil. Furthermore, the cleansing composition may include a combination of cera alba, hydrogenated castor oil, cetearyl alcohol, and glyceryl stearate, The naturally occurring wax may comprise cera alba and cetearyl alcohol. The naturally occurring wax may comprise hydrogenated caster oil and cetearyl alcohol. The naturally occurring wax may consist of cera alba and cetearyl alcohol. The naturally occurring wax may consist of hydrogenated caster oil and cetearyl alcohol. Other combinations of naturally occurring waxes are contemplated herein.

When hydrogenated castor oil and cetearyl alcohol are used in combination, the cetearyl alcohol may be present in a higher weight amount than the hydrogenated castor oil. When cera alba and cetearyl alcohol are used in combination, the cetearyl alcohol may be present in a higher weight amount than the cera alba. When cetearyl alcohol is used in combination with a mixture of hydrogenated castor oil and cera alba, the cetearyl alcohol may be present in a higher weight amount than the hydrogenated castor oil/cera alba mixture.

The cleansing composition includes an oil, where the oil is liquid at 20 °C, and wherein at least about 50% by weight of the oil is caprylic/capric triglyceride or isopropyl myristate or a mixture thereof. The oil may be present in a total amount of from about 30% to about 65% by weight of the cleansing composition, or from about 35% to about 55% by weight of the cleansing composition, or from about 35% to about 45% by weight of the cleansing composition, preferably from about 39% to about 40% by weight of the cleansing composition. The total weight of the oil present in the cleansing composition may be greater than the total weight of the naturally occurring wax. Oils may include, for example, caprylic/capric triglyceride, isopropyl myristate, isopropyl palmitate, ethylhexyl palmitate, and combinations thereof. The oil may comprise isopropyl myristate, or the oil may consist of isopropyl myristate.

The cleansing composition includes a surfactant, which may include nonionic, anionic, or amphoteric surfactants, or may include combinations of surfactants. The surfactant may be present in a total amount of from about 5% to about 30% by weight of the cleansing composition, or from about 10% to about 20% by weight of the cleansing composition, or from about 16% to about 25% by weight of the cleansing composition. Surfactants useful in the cleansing composition include, for example, polysorbate 80, polyglyceryl-3 diisostearate, PEG-8 caprylic/capric glycerides, and combinations thereof. For example, the surfactant may comprise polysorbate 80, or the surfactant may consist of polysorbate 80. It is desired that the surfactant used in the cleansing composition be capable of serving as an emulsifier or emulsifying agent, thereby allowing the balm to become emulsified when combined with water (e.g., during rinsing of the balm off of the skin). It is desired that the balm begins emulsifying within ten seconds after contact with water, or within seven seconds after contact with water, or within five seconds after contact with water, to allow for quick and easy rinsing of the balm from the skin.

The cleansing composition may include an emollient, where the emollient or emollient blend is useful in cleansing the skin. As used herein, "emollients" refer to materials used for cleansing the skin, hair, and eye lashes, the prevention or relief of dryness, or for the protection of the skin. Examples of emollients useful herein include, but are not limited to, linear or branched chained hydrocarbon emollients, such as decane, isohexadecane, dodecane, tridecane, tetradecane, squalene, hexadecane, or isoparaffins; hydrophobic compounds such as vegetable oils; mineral oils (e.g., petrolatum); fatty esters (e.g., isopropyl palmitate, C12-C15 alkyl benzoate) including those fatty esters of glycerol, linear or branched alkyl esters; stearic esters, such as isostearates, including isopropyl isostearate, decyl isostearate (including simple isostearates and highly mono-branched decyl isostearates, which is an ester formed from an isostearic acid having an increased proportion of mono-branched molecules compared to simple isostearic acid), and hexyl isostearate; and combinations thereof.

The cleansing composition may include one emollient or may include more than one emollient. The emollient(s) may be present in a total amount of from about 3% to about 15% by weight of the cleansing composition, or from about 5% to about 10% by weight of the cleansing composition, or from about 7% to about 8% by weight of the cleansing composition. Each individual emollient may independently be present in an amount of from about 0.1% to about 5% by weight of the cleansing composition, or from about 1% to about 5% by weight of the composition, or from about 1.4% to about 3.5% by weight of the composition, or from about 1.8% to about 2.8% by weight of the composition.

The composition may include a blend of emollients, such as that described in U.S. Patent Application Number 2019/0336407, the contents of which are incorporated by reference herein. This emollient blend includes a first stearic ester, a second stearic ester, and a branched hydrocarbon in a weight ratio of about 2:2:1, or alternatively a weight ratio of about 1:1:1.1 to about 1:1:3. By way of example, the first stearic ester may be decyl isostearate, the second stearic ester may be isopropyl isostearate, and the branched hydrocarbon may be isohexadecane. Preferably, the emollient may comprise or consist of a blend of isohexadecane, decyl isostearate and isopropyl isostearate. Other emollients are useful in the cleansing composition, as described above.

The cleansing composition may be free or substantially free of antimicrobial preservatives. The cleansing composition may include antioxidants, such as tocopheryl acetate. If used, antioxidants may be present in an amount of from about 0.05% to about 0.5% by weight of the cleansing composition, or from about 0.1% to about 0.25% by weight of the cleansing composition, or about 0.1% by weight of the cleansing composition.

The cleansing composition may additionally include one or more humectants, such as panthenol or glycerin. Humectants, if used, may be present in an amount less than about 1% by weight, or less than about 0.5% by weight, or less than about 0.25% by weight.

The cleansing composition may additionally include one or more fragrances or fragrance blends, including essential oils or other fragrance ingredients. If used, fragrances are present in a total weight of from about 0.001% to about 0.5% by weight of the composition, or from about 0.01% to about 0.2% by weight of the composition, or about 0.1% by weight of the composition. It is desired that the balm be free or substantially free of added colorants or dyes. It is desired that the balm be opaque and white, avoiding having a grayish color.

The cleansing compositions are stable and semisolid at room temperature, as described above. It is desired that the compositions described herein be contained in a suitable container, which may be opened and closed repeatedly, allowing the user to extract some of the balm for use on the skin. Suitable containers include jars, bottles, tubes, sachets, and the like, with bottles and jars having rigid sides being preferred.

The present invention includes not only the cleansing composition described herein, but also includes a method of using the cleansing composition. In use, the user opens the container, extracts some of the balm composition with the fingers, hand, or using a tool such as a spoon or stick, and applies the balm composition to the desired area of the skin. For example, the user may apply the balm composition to the face, including ocular area. The balm may be rubbed onto the target area of the skin, such as with the hand or fingers of the user, or with a sponge, cotton ball, wipe, or other apparatus. The cleansing composition may be applied while the skin is wet or dry. The user may apply any desired amount, such as from 1 cm³ to 20 cm³, or more, as needed to apply to the desired area of the skin or face.

When the user has completed applying the product to the target area and rubbing it sufficiently, the user may rinse the product off the skin. Rinsing may include splashing water and rubbing with the hands or cloth, or may include using a wet product, such as a washcloth. The user applies water to the skin, and rubs with the hands or the cloth, allowing the applied balm to emulsify in the presence of the water. As noted above, the balm of the present invention begins forming an emulsion when mixed with water within about 10 seconds, 7 seconds, or 5 seconds, allowing the user to prepare the emulsified balm quickly and easily and rinse it from the skin. Once the emulsion is formed, the user then may wash their face as per usual practice, removing the emulsified balm, along with any dirt, oil, makeup, or other materials that it has cleansed from the skin. The method described herein may be practiced in the absence of any component, ingredient or step not specifically disclosed herein, or may include additional components or steps that are not expressly disclosed herein.

The compositions described herein are useful in removing various cosmetic products from the skin of users, and in particular, are useful in removing at least one of: mascara, foundation, lipstick and eyeliner efficiently. Mascara is a notoriously difficult cosmetic material to remove from skin, as it deposits high levels of film formers and includes a relatively high level of hydrophobic materials. Further, mascara includes a high level of carbon dark pigments, giving it a dark color. In addition, since mascara is typically applied on the eyes or eyelashes, cleaning requires a gentle, low pressure and non-planar application. Eyeliner presents its own difficulties in removal, as it includes titanium dioxide, iron oxide, mica, silica and higher concentration of pigments/colorants, which may be more difficult to remove. In addition, as with mascara, eyeliner is often applied to the eyes or eye region, and cleansing requires a gentle, lower pressure and non-planar application. Foundation provides some difficulty as well, as it is typically an emulsion and may include silicones, which cause it to remain on the skin. Cosmetics today are geared towards a "24-hour" use, where the cosmetic remains on the skin of the user for an extended period of time. These "long-lasting" and waterproof cosmetics have a high transfer resistance, making them difficult to remove.

It is desired to provide a composition that effectively and sufficiently removes enough of at least one, at least two, or all three of the aforementioned cosmetic materials. Removal of enough of a cosmetic includes removing at least about 95% of the cosmetic from the skin, or at least about 99% of the cosmetic from the skin, or at least about 99.9% of the cosmetic from the skin. Further, it is desired to provide a composition that does not leave an oily or greasy residue on the surface of the skin after rinsing, since such oily cleansers may be difficult to remove and leave the user with an unclean feeling. In addition, it is beneficial that the cleansing composition provide low or no irritation to the skin, particularly the eyes. A product that has little to no visual defects, such as crystals, cracks or discoloration is preferred. Further, being able to provide a composition that is capable of maintaining a semisolid state at room temperature, while also allowing the user to remove some of the product and apply to the skin, is desired. The present invention provides a cleansing composition that has desirable characteristics defined herein, while providing suitable cleansing capabilities as well.

As noted above, the present invention is directed to compositions that can be used to clean a variety of cosmetics off the surface of a user's skin effectively and efficiently. The inventive compositions should be capable of removing, for example, foundation, lipstick, eyeshadow, lipliner, eyebrow, pencil, pomade, blush mascara, concealer, highlighter and/or eyeliner efficiently. Further, the inventive compositions may be substantially free of silicones, and more desirably entirely free of silicones.

A method of making a cleansing composition in the form of a balm includes the steps of combining: a naturally occurring wax, wherein the naturally occurring wax comprises at least about 10% by weight of the cleansing composition of cetearyl alcohol, and at least about 6.5% by weight of the cleansing composition of either hydrogenated castor oil or cera alba or a mixture thereof; an oil in the form of a liquid at 20 °C, wherein at least about 50% by weight of the oil is caprylic/capric triglyceride or isopropyl myristate or a mixture thereof; a surfactant; and optionally an emollient together to form a balm that is stable at room temperature. The steps of combination may be rearranged, and in one method, the steps include first providing a liquid oil or combining the liquid oils, if more than one oil is used, to form an oil base. Then, the naturally occurring wax or waxes are added together with the oil(s) to form a wax and oil composition. The formulation may then be heated, if desired, to melt the naturally occurring wax or waxes. The wax and oil composition may be heated to a temperature of about up to 60 °C, forming a heated wax and oil composition, which is in the form of a flowable liquid.

The surfactant or surfactants, any emollient or emollients, any antioxidants, humectants, and fragrances are then added to the heated wax and oil composition. The combined blend is then mixed, which can be achieved with any mixing apparatus and mixing rate desired. The composition, having been heated, is still in a flowable liquid form, and may be deposited into a container, such as a jar or bottle. The composition can be filled to any desired level in the container, forming a filled container. The filled container is allowed to cool, such as to room temperature, and the cleansing composition becomes stable and semisolid in the form of a balm. The container may be sealed and provided to a consumer. In use, the consumer, simply opens the container, such as by removal of a lid or cap, dispenses some of the cleansing composition, and applies it to the skin. The consumer should close the container after use.

The hardness value refers to the amount of force required to penetrate through the outer surface of the cleansing composition in the form of a balm in its stored state. The greater the amount of force required, the "harder" the balm is and the greater the hardness value, and vice versa. When stored, such as prior to use, the cleansing composition should be a semisolid material, which is not free flowing but also not too hard to be used by a consumer. The user should be capable of removing a portion of the composition, such as with the fingers or a tool, and applying to the skin surface, where application warms the composition sufficiently to make it less hard, thereby allowing application. As can be understood, the hardness value of the cleansing composition in the form of a balm prior to use is an important indicator of whether a product is suitable for use in this form. If a product is too hard, it would not be easily applied to the skin by the user, and if the product is too soft, it would not be sufficiently maintained as a balm product.

The hardness value may be measured using a penetration test with a texture analyser. For example, the hardness value may be measured using a Texturometer (TA.XT-plus, from Stable Micro Systems, UK) at 20 °C, the Texturometer being equipped with a force captor and a cylindrical probe, 2mm diameter. The probe penetrates the product through a vertical displacement and a sensor measures the maximal positive force during the penetration expressed in grams. Each measurement is repeated 4 to 6 times and a mean value for the hardness value is obtained.

The present invention may be better understood through the following examples, which are exemplary in nature and not intended to be limiting to any specific combination of elements.

### Examples

Examples E1-E5 are sample compositions that provide suitable stability and effectiveness in a cleansing composition in the form of a balm. Examples C1-C7 are sample compositions that did not provide a suitable cleansing composition in the form of a balm, for one or more reasons that are identified below.

Each example below includes a combination of one or more oils (which are liquid at 20 °C), one or more waxes (which are solid at room temperature, e.g. approximately 20 °C), one or more surfactants and optionally one or more emollients, with an optional fragrance added to each of the Example cleansing compositions. Each cleansing composition is intended to be maintained in a semisolid state at room temperature, where it can be removed from a container and applied to the skin surface of the user.

Each sample was evaluated for its color, its hardness value, its ability to emulsify at rinsing, and other aesthetic observations. While an optimal composition has highest scores for each category, it is not necessary that a composition have the highest rated scores for each of these categories. Taken together, the categories help determine whether the composition is sufficient as a cleansing balm or not.

Color means the color of and ability of light to travel through the cleansing composition, with opaque whiteness being the desired traits of the composition. The color and opaqueness of each composition was rated, and scores reflected below. A score of W means that the color was white or extremely close to white, and was opaque. A score of SG means that the formulation was slightly gray or slightly translucent. A score of G means that the formulation was gray, translucent or yellowish.

The hardness value refers to the amount of force required to penetrate through the outer surface of the cleansing composition in the form of a balm in its stored state. The greater the amount of force required, the "harder" the balm is and the greater the hardness value, and vice versa. When stored, such as prior to use, the cleansing composition should be a semisolid material, which is not free flowing but also not too hard to be used by a consumer. The user should be capable of removing a portion of the composition, such as with the fingers or a tool, and apply to the skin surface, where application warms the composition sufficiently to make it less hard, thereby allowing application. Each composition was rated on its hardness value, in terms of suitability for this purpose. As can be understood, the hardness value of the cleansing composition in the form of a balm prior to use is an important indicator as to whether a product was suitable for use in this form. If a product is too hard, it would not be easily applied to the skin by the user, and if the product is too soft, it would not be sufficiently maintained as a balm product.

The hardness value was measured using a penetration test with a Texturometer (TA.XT-plus, from Stable Micro Systems, UK) at approximately 20 °C. The Texturometer was equipped with a force captor and a cylindric probe, 2mm diameter. The probe penetrates the product through a vertical displacement and the sensor measures the maximal positive force during the penetration expressed in grams. Each measurement was repeated 4 to 6 times and the mean values are indicated in the Tables below.

Compositions were also evaluated for the ability to be rinsed off the skin after being applied by the user. This is referred to as emulsifying at rinse, and specifically refers to the capacity of the composition to create an emulsion when water is added, allowing the balm to rinse off of the skin to which it has been applied. A score of + indicates that the composition sufficiently emulsified in the presence of water, with a score of + indicating sufficient emulsifying, a score of ++ indicating better emulsifying, and a score of +++ indicating the best emulsifying properties. A score of - indicates that the composition did not emulsify properly, and thus was not considered acceptable for use as a cleansing balm. Emulsifying at rinse was measured by human examination, with the results describing the individual's personal evaluation of the sample's rinsing ability.

Finally, each composition was observed for other characteristics. For example, the composition may have been observed for its texture, its stability, its color, or other properties. A sufficiently stable composition should have a smooth texture, having little to no visible cracks, with no visible crystals or granular texture, and should not form droplets on the surface thereof or within the body of the composition. Visual aesthetics were determined by viewing only with the eyes and without the aid of instruments such as a microscope.

**Table 1: Compositions E1-E5**

| | | E1 | E2 | E3 | E4 | E5 |
|---|---|---|---|---|---|---|
| Oil (liquid) | Capry lic/Capric Triglyceride | 41.17 | 47.37 | 0 | 59 | 46 |
| | Isopropyl Myristate | 0 | 0 | 37.37 | 0 | 0 |
| | Isopropyl Palmitate | 0 | 0 | 0 | 0 | 0 |
| | Ethylhexyl Palmitate | 0 | 0 | 0 | 0 | 0 |
| Wax (solid) | Cera Alba | 0 | 0 | 14 | 0 | 0 |
| | Hydrogenated Castor Oil | 9.6 | 11 | 0 | 6.50 | 10 |
| | Cetearyl Alcohol | 12.00 | 14 | 25 | 10.00 | 16 |
| | Glyceryl Stearate | 9.6 | 0 | 0 | 4.50 | 8 |
| Surfactant | Polysorbate 80 | 20 | 20 | 16 | 20 | 20 |
| | Polyglyceryl-3 Diisostearate | 0 | 0 | 0 | 0 | 0 |
| | PEG-8 Caprylic/capric Glycerides | 0 | 0 | 0 | 0 | 0 |
| Emollient | Isohexadecane | 1.4 | 1.4 | 1.4 | 0 | 0 |
| | Decyl Isostearate | 2.8 | 2.8 | 2.8 | 0 | 0 |
| | Isopropyl Isostearate | 2.8 | 2.8 | 2.8 | 0 | 0 |
| | Aqua | 0.00 | 0 | 0 | 0 | 0 |
| Antioxidant | Tocopheryl Acetate | 0.1 | 0.1 | 0.1 | 0 | 0 |
| Humectant | Panthenol | 0.33 | 0.33 | 0.33 | 0 | 0 |
| | Fragrance | 0.2 | 0.2 | 0.2 | 0 | 0 |
| | Hardness value (gram) | 123.4 | 108.2 | 56.9 | 27.5 | 42.9 |
| | Color | W | W | W | G | G |
| | Emulsify at Rinse | +++ | +++ | ++ | +++ | +++ |
| | Observation | Stable | Stable | Stable | Stable | Stable |

Examples E1-E3 each were white and opaque, had a sufficient hardness value, were capable of being emulsified in the presence of liquid and appeared stable visually. E1 had the highest hardness value, and its sensory perception (sufficiently solid and capable of being applied to the skin once removed from the container) was inferior to E2 although still acceptable. Regarding the hardness value, E3, E4 and E5 were preferred. Examples E4 and E5 had a sufficient hardness value, were capable of being emulsified in the presence of liquid, and appeared stable visually, however these samples appeared gray/translucent in color. Although the color of Examples E4 and E5 was not ideal, these compositions passed the hardness value and emulsifying tests, and visually were deemed stable and free of visual crystals or phase separation. Thus, examples E4 and E5 were also deemed successful as cleansing balms.

**Table 2: Compositions C1-C4**

| | | C1 | C2 | C3 | C4 |
|---|---|---|---|---|---|
| Oil (liquid) | Caprylic/Capric Triglyceride | 53 | 65 | 52.37 | 5 |
| | Isopropyl Myristate | 0 | 0 | 0 | 0 |
| | Isopropyl Palmitate | 0 | 0 | 0 | 47.42 |
| | Ethylhexyl Palmitate | 0 | 0 | 0 | 0 |
| Wax (solid) | Cera Alba | 0 | 0 | 0 | 10 |
| | Hydrogenated Castor Oil | 3 | 10 | 6 | 0 |
| | Cetearyl Alcohol | 16 | 4 | 8 | 10 |
| | Glyceryl Stearate | 8 | 1 | 6 | 0 |
| Surfactant | Polysorbate 80 | 20 | 20 | 20 | 0 |
| | Polyglyceryl-3 Diisostearate | 0 | 0 | 0 | 10 |
| | PEG-8 Capry lic/capric Glycerides | 0 | 0 | 0 | 7.35 |
| Emollient | Isohexadecane | 0 | 0 | 1.4 | 1.4 |
| | Decyl Isostearate | 0 | 0 | 2.8 | 2.8 |
| | Isopropyl Isostearate | 0 | 0 | 2.8 | 2.8 |
| | Aqua | 0 | 0 | 0 | 2.5 |
| Antioxidant | Tocopheryl Acetate | 0 | 0 | 0.1 | 0.1 |
| Humectant | Panthenol | 0 | 0 | 0.33 | 0.33 |
| | Fragrance | 0 | 0 | 0.2 | 0.3 |
| | Hardness value | 16.7 | 21.3 | 29.5 | 8.5 |
| | Color | SG | G | w | SG |
| | Emulsify at Rinse | +++ | +++ | +++ | + |
| | Observation | stable | stable | Crystals | Phase separation Granular texture |

**Table 3: Compositions C5-C7**

| | | C5 | C6 | C7 |
|---|---|---|---|---|
| Oil (liquid) | Capry lic/Capric Triglyceride | 5 | 5 | 0 |
| | Isopropyl Myristate | 0 | 0 | 0 |
| | Isopropyl Palmitate | 34.92 | 39.92 | 6 |
| | Ethylhexyl Palmitate | 0 | 0 | 39.37 |
| Wax (solid) | Cera Alba | 15 | 15 | 15 |
| | Hydrogenated Castor Oil | 0 | 0 | 0 |
| | Cetearyl Alcohol | 20 | 15 | 20 |
| | Glyceryl Stearate | 0 | 0 | 0 |
| Surfactant | Polysorbate 80 | 0 | 0 | 12 |
| | Polyglyceryl-3 Diisostearate | 10 | 10 | 0 |
| | PEG-8 Caprylic/capric Glycerides | 7.35 | 7.35 | 0 |
| Emollient | Isohexadecane | 1.4 | 1.4 | 1.4 |
| | Decyl Isostearate | 2.8 | 2.8 | 2.8 |
| | Isopropyl Isostearate | 2.8 | 2.8 | 2.8 |
| | Aqua | 0 | 0 | 0 |
| Antioxidant | Tocopheryl Acetate | 0.1 | 0.1 | 0.1 |
| Humectant | Panthenol | 0.33 | 0.33 | 0.33 |
| | Fragrance | 0.3 | 0.3 | 0.2 |
| | Hardness value | 64.4 | 41.3 | 47.4 |
| | Color | SG | SG | SG |
| | Emulsify at Rinse | - | - | - |
| | Observation | Phase separation; Granular texture | Phase separation; Granular texture | Phase separation |

Compositions C1-C7 were prepared and tested for the aforementioned characteristics. As can be seen in the Tables 2 and 3 above, each failed in one or more of the measured criteria. Examples C1, C2 and C4 were not hard enough to be considered suitable cleansing balms. Moreover, Example C4 visually demonstrated phase separation and a granular texture, thus failing the visual aesthetic test. Example C3 failed the visual aesthetic test, in that it showed crystal formation. Examples C5-C7 each had an appropriate hardness value but failed the emulsifying test, and visually all showed phase separation, with Examples C5 and C6 also showing a granular texture.

It is noted that Example C3 passed the hardness value, color, and emulsifying criteria, but since the visual characteristics and texture requirements are considered important in the formation of a useful cleansing balm, the formation of visible crystals was deemed a failure. The lower levels of hydrogenated castor oil and cetearyl alcohol may have been a factor in the formation of visible crystals.

## Claims

1. A cleansing composition in the form of a balm, comprising:
a. a naturally occurring wax, wherein the naturally occurring wax comprises at least about 10% by weight of the cleansing composition of cetearyl alcohol, and at least about 6.5% by weight of the cleansing composition of either hydrogenated castor oil or cera alba or a mixture thereof;
b. an oil, the oil being liquid at 20 °C, wherein at least about 50% by weight of the oil is caprylic/capric triglyceride or isopropyl myristate or a mixture thereof;
c. a surfactant; and
d. optionally an emollient;
wherein the cleansing composition is substantially free of water.

2. The cleansing composition of claim 1, further comprising a humectant and/or an antioxidant.

3. The cleansing composition of claim 1 or claim 2, wherein the cleansing composition has a hardness value of from about 25 grams to about 125 grams at 20 °C, optionally from about 25 grams to about 60 grams at 20 °C.

4. The cleansing composition of any preceding claim, wherein the composition begins forming an emulsion in the presence of water within ten seconds.

5. The cleansing composition of any preceding claim, wherein the composition is substantially free of synthetic wax.

6. The cleansing composition of any preceding claim, wherein the composition is substantially free of antimicrobial preservatives.

7. The cleansing composition of any preceding claim, wherein the total amount of naturally occurring wax present is from about 16.5% to about 50% by weight of the cleansing composition, optionally from about 16.5% to about 40% by weight of the cleansing composition, optionally from about 21% to about 39% by weight of the cleansing composition.

8. The cleansing composition of any preceding claim, wherein the naturally occurring wax comprises cetearyl alcohol and hydrogenated castor oil.

9. The cleansing composition of any of claims 1-7, wherein the naturally occurring wax comprises cetearyl alcohol and cera alba.

10. The cleansing composition of any preceding claim, wherein the naturally occurring wax further comprises glyceryl stearate.

11. The cleansing composition of any preceding claim, wherein the cetearyl alcohol is present in a weight amount greater than the weight amount of the hydrogenated castor oil or cera alba or mixture thereof.

12. The cleansing composition of any preceding claim, wherein the total amount of surfactant present is from about 5% to about 30% by weight of the cleansing composition.

13. The cleansing composition of any preceding claim, wherein the surfactant comprises polysorbate 80, optionally wherein the surfactant consists of polysorbate 80.

14. The cleansing composition of any preceding claim, wherein the composition comprises an emollient.

15. The cleansing composition of claim 14, wherein the total amount of emollient present is from about 3% to about 15% by weight of the cleansing composition, optionally from about 5% to about 10% by weight of the cleansing composition.

16. The cleansing composition of claim 14 or claim 15, wherein the emollient comprises at least one stearic acid and at least one branched hydrocarbon.

17. The cleansing composition of any of claims 14 to 16, wherein the emollient comprises isohexadecane, decyl isostearate and isopropyl isostearate.

18. The cleansing composition of any preceding claim, wherein the total weight of the oil present is greater than the total weight of the naturally occurring wax.

19. The cleansing composition of any preceding claim, wherein the oil comprises isopropyl myristate.

20. The cleansing composition of any of claims 1 to 7, 9 and 12 to 19, wherein the oil consists of isopropyl myristate, the naturally occurring wax consists of cera alba and cetearyl alcohol, and
wherein the cetearyl alcohol is present in an amount greater than the cera alba.

21. The use of a cleansing composition of any preceding claim to remove at least one of dirt, oil, grease or makeup from skin.

22. A method of cleansing skin comprising:
(i) applying the cleansing composition of any of claims 1 to 20 to a region of skin of a user;
(ii) subsequently applying water to the region of skin, thereby causing the cleansing composition to emulsify; and
(iii) rinsing the region of skin to remove the emulsified cleansing composition.

23. The method of claim 22, wherein, the emulsified cleansing composition remains on the region of skin for a period of time, wherein the period of time is from about 5 seconds to about 5 minutes.
